# EUROPÄISCHE PATENTSCHRIFT

(11) **EP 1 551 373 B1**
(45) Veröffentlichungstag und Bekanntmachung des Hinweises auf die Patenterteilung: **12.11.2014**
(21) Anmeldenummer: 03808702.9
(22) Anmeldetag: 30.09.2003
(51) Int. Cl.: A61K 9/19, A61K 8/02, C08L 5/04

(54) **VERWENDUNG VON FORMKÖRPERN ZUR ÄUSSEREN ANWENDUNG**
USE OF MOLDED BODIES FOR EXTERNAL APPLICATION
UTILISATION D'ELEMENTS MOULES POUR UNE APPLICATION EXTERNE

(30) Priorität: 16.10.2002 DE 10248314; 17.04.2003 DE 10317982
(43) Veröffentlichungstag der Anmeldung: 13.07.2005
(73) Patentinhaber: MedSkin Solutions Dr. Suwelack AG, 48727 Billerbeck (DE)
(72) Erfinder: FRAHLING, Stefan, 48727 Billerbeck (DE); MALESSA, Ralf, 45134 Essen (DE)
(74) Vertreter: Gille Hrabal
(86) Internationale Anmeldenummer: PCT/EP2003/010853
(87) Internationale Veröffentlichungsnummer: WO 2004/035023

(56) Entgegenhaltungen:
- EP-A- 0 888 769
- DE-A- 4 328 329
- US-A- 4 758 598
- US-A- 5 578 307

## Beschreibung

Die Erfindung betrifft die Verwendung eines Formkörpers, enthaltend mindestens einen Gerüstbilder, wobei Proteine ausgenommen sind, gegebenenfalls einen oder mehrere Wirkstoffe sowie gegebenenfalls einen oder mehrere Hilfsstoffe, der dadurch gekennzeichnet ist, dass er die geometrische Form einer Kugel mit einem Durchmesser von mindestens 5 mm und eine Auflösungsgeschwindigkeit von weniger als 1 Minute, entsprechend der Methode zur Messung der "Zerfallszeit von Tabletten und Kapseln" mittels PharmEU Testapparatur, aufweist, zur äußeren Anwendung, ein Verfahren zur Herstellung der genannten Formkörper sowie neue Formkörper.

Aus den deutschen Patentschriften DE 42 01 172, DE 42 01 173 sowie DE 42 01 179 sind Pellets zur pharmazeutischen oder kosmetischen Anwendung beschrieben, die Gerüstbildner auf der Basis von Proteinen aufweisen. Die Pellets dienen Insbesondere zur Herstellung oraler Arznelmtttelformullerungen. Die Pellets werden hergestellt, In dem Dispersionen aus den Protein-Gerüstblldnern und gegebenenfalls kosmetischen oder pharmazeutischen Wirkstoffen in tiefkalte Inerte Flüssigkeiten, vorzugsweise flüssigen Stickstoff, eingetropft werden und die gefrorenen Pellets anschließend abgetrennt und gefriergetrocknet werden. Um unter diesen Bedingungen Pellets zu bilden, Ist jedoch die Anwesenheit von Protein-Gerüstbildnern, Insbesondere Kollagen bzw. Kollagen-Derivaten erforderlich, da nur die genannten Protein-Gerüstbildner unter diesen Bedingungen stabile Pellets ausbilden können. Dies hängt vermutlich mit den speziellen Intermolekularen Wechselwirkungen der Proteinmoleküle zusammen.

Die Größe der nach den genannten Deutschen Patentschriften hergestellten Pellets wird Im allgemeinen Teil der Beschreibungen mit 0,2 bis 12 mm angegeben. In den Beispielen liegt die maximal erzielte Größe Jedoch lediglich bei 4 mm. Dabei handelt es sich um eine Durchschnittsgröße, d.h. die Pellets unterliegen mehr oder weniger starken Größenschwankungen.

Insbesondere für die äußere kosmetische Anwendung werden Jedoch relativ große Formkörper einheitlicher Form und Größe bevorzugt. Der Endverbraucher kann solche Formkörper einfach handhaben. Pulver, bzw. kleine Pellets oder ungleichmäßig geformte Formkörper sind für diese Zwecke in der Regel ungeeignet. Man Ist bestrebt, Formkörper einer solchen Größe bereitzustellen, die es erlauben pro Anwendung eine Dosierungsform zu benutzen, Außerdem vermitteln größere Formkörper, die z.B. farblich gestaltet werden können, auch einen stärkeren ästhetischen Eindruck.

Die Anwendung von Protein-Gerüstbildner ist außerdem in einigen Fällen nicht bevorzugt. So ziehen einige Endverbraucher zunehmend die Anwendung rein pflanzlicher Produkte insbesondere in der Kosmetik vor, Die Gründe hierfür resultieren dabei unter anderem aus grundsätzlichen, ethischen Erwägungen.

Die Aufarbeitung von Proteinen erfordert weiterhin in der Regel komplizierte Aufbereitungsschritte. Ferner sind die Eigenschaften der Protein-Gerüstbildner bei der äußeren Anwendung auf der Haut in Ihrem Eigenschaftsspektrum In vielen Fällen zu beschränkt, da sie stets aus denselben Aminosäuren zusammengesetzt sind.

Es besteht daher zunehmend der Wunsch nach kosmetischen oder pharmazeutischen Formformullerungen, insbesondere Formkörpern zur äußeren Anwendung, die nicht auf Protein-Basis beruhen. Will man jedoch Gerüstbildner auf pflanzlicher Basis, wie z.B. Alginate, nach dem in den vorstehend genannten Patentschriften verwendeten Verfahren verarbeiten, so stellt man überraschend fest, dass sich auf diese Weise keine Formkörper mit regelmäßigen Formen, insbesondere in ausreichender Größe und mit ausreichender Löslichkeit, wie sie für die äußeren Anwendung erforderlich sind, herstellen lassen.

Zahlreiche Veröffentlichungen sind mit der Herstellung oraler Formulierungen befasst, deren Herstellung einen Gefriertrocknungsschritt beinhaltet. So sind aus der EP-A-0352190 aus festen und porösen Einheitsformen bestehende Zusammensetzungen bekannt, die Mikro- oder Nanoteilchen umfassen. Diese werden unter anderem so hergestellt, dass Pasten in Hohlräume einer Form gegeben werden und anschließend eine Lyophilisierung durchgeführt wird. Die Aufgabenstellung der EP-A-0352190 bestand darin, orale Formulierungen mit einer Geschmacksmaskierung zu finden, die sich in Wasser schnell auflösen. Eine topische Anwendung der Formulierungen wird nicht erwähnt. Ähnlich offenbart die EP-A-0399902 durch Gefriertrocknung hergestellte Formulierungen, die ebenfalls der Bereitstellung geschmacksmaskierter schnell löslicher Arzneimittelformulierungen dienen, Aus der US-A-4758598 sind durch Gefriertrocknung hergestellte Formulierungen bekannt, die ebenfalls ausschließlich zur oralen Anwendung dienen. Aus der US-A-4079018 sind lösliche Trägermaterlailen zur Verabreichung von Arzneimitteln zur oralen Anwendung bekannt. Aus der US 4 695 463 sind Verabreichungssysteme mit kontrollierter Freisetzung zur oralen Verabreichung beschrieben. Ferner sind aus der EP-A-0412449 gefriergetrocknete Zusammensetzungen bekannt, die jedoch keine regelmäßige Geometrie aufweisen.

Die DE 4328329 A1 offenbart schlchtförmige gefriergetrocknete Biomatrices mit einer Dicke von 1 mm. Die hier offenbarten schichtförmigen Biomatrices werden nach Befeuchten als Gesichtsmaske verwendet oder in Gelform überführt und in die Haut einmassiert.

Die EP 0888769 A1 offenbart ebenfalls schichtförmige lyophilisierte kosmetische Zubereitungen basierend auf Alginaten und Wirkstoffen zur Verwendung als Masken zum Auftragen auf die Haut für einen Zeitraum von 10 bis 15 Minuten.

Keine der vorstehenden Entgegenhaltungen offenbart jedoch die Möglichkeit der Herstellung von relativ großformatigen regelmäßigen Formkörpern, die sich bei der äußeren Anwendung auf der Haut leicht lösen und in der Lage sind, als Wirkstoffträger zu fungieren.

Die Aufgabe der vorliegenden Erfindung bestand somit darin eine neue Verwendung wirkstoffhaltiger Formkörper, die Gerüstbildner aufweisen, die nicht auf Proteinen basieren, für die äußere Anwendung bereitzustellen. Die Formkörper sollten dabei relativ groß sein, eine ausreichende Kohäslon, d.h. mechanische Festigkeit, aufweisen, regelmäßig geformt sein, sich bei der Anwendung auf der Haut leicht lösen und zu einem angenehmen Anwendungsgefühl führen. Welterhin sollten diese Formkörper geeignet sein, verschiedene Wirkstoffe wie Insbesondere kosmetische Wirkstoffe und therapeutische bzw. pharmazeutische Wirkstoffe aufzunehmen und als Träger hierfür fungieren.

Die Erfindung stellt somit die Verwendung eines Formkörpers bereit, enthaltend mindestens einen Gerüstbildner, wobei Proteine ausgenommen sind, gegebenenfalls einen oder mehrere Wirkstoffe sowie gegebenenfalls einen oder mehrere Hilfsstoffe zur äußeren Anwendung.

Unter einem Formkörper im Sinne der Erfindung versteht man einen regelmäßig geformten geometrischen Körper In Form einer Kugel Quader, Pyramiden, Sterne aber auch natürlichen Formen nachgebildete Formkörper wie z.B. solche In der Form von Tieren, wie z.B. Meerestieren, wie z.B. Seesterne, Meeresfrüchte, wie Muscheln, etc. Pflanzen und Pflanzenteilen, wie Blätter etc. Sind nach dem welter unten beschriebenen Verfahren zur Herstellung der erfindungsgemäß verwendeten Formkörper zugänglich. Erfindungsgemäß Ist auch eine Mehrzahl der genannten Formkörper in einem Behältnis umfasst. Die Formkörper können einzeln abgepackt sein. Bevorzugt liegt jedoch Insbesondere in der kosmetischen Anwendung eine Mehrzahl der Formkörper nebeneinander in Kontakt In einem Behältnis vor.

Die Volumina der verwendeten Formkörper sind aufgrund des Verfahrens Ihrer Herstellung an sich nicht beschränkt. Zweckmäßig liegen die Volumina bevorzugt bei mindestens etwa 0,1 cm³, bevorzugt 0,3 cm³, bevorzugter mindestens etwa 0,5 cm³, noch bevorzugter mindestens etwa 0,8 cm³, Nach oben werden die verwendeten Volumina zweckmäßig auf bis zu etwa 6 cm³, bevorzugt bis zu etwa 5 cm³, bevorzugter bis zu etwa 4 cm³ beschränkt. Die Größe der Formkörper wird unter anderem durch den Ort der äußeren Anwendung der Formkörper bestimmt. So ermöglicht die Anwendung auf größeren Körperflächen oder den Haaren (z.B. der direkte Auftrag der angefeuchteten Formkörper auf dem Rücken etc., oder der Einsatz als Badezusatz) den Einsatz größerer Formkörper, wohingegen bei der Anwendung auf kleineren Körperpartien (z.B. Wange etc.) kleinere Formkörper bevorzugt sind.

Der Durchmesser eines erfindungsgemäßen Formkörpers (maximaler Abstand zwischen zwei Punkten In einem Formkörper In Form einer Kugel liegt zweckmäßig bei mindestens 5 mm, bevorzugter mindestens etwa 7 mm, noch bevorzugter mindestens etwa 8 mm bis hln zu zweckmäßig etwa 60 mm, bevorzugt etwa 50 mm, bevorzugter etwa 40 mm, noch bevorzugter etwa 30 mm.

Ein besonders bevorzugter Formkörper weist eine im wesentlichen kugelförmige Geometrie auf, wobei der Durchmesser der Kugel zwischen 3 bis 30 mm, bevorzugt zwischen 5 und 20 mm, bevorzugter zwischen 7 und 15 mm, noch bevorzugter zwischen 8 und 13 mm liegt.

Der erfindungsgemäß verwendete Formkörper enthält mindestens einen Gerüstbildner, wobei Proteine ausgenommen sind. Bei dem Gerüstbildner handelt es sich Im allgemeinen um sogenannte Hydrokolloide, d.h. (teilweise) wasserlösliche, natürliche oder synthetische Polymere, die in wässrigen Systemen Gele bzw. viskose Lösungen bilden. Die Gerüstbildner werden zweckmäßig ausgewählt aus Polysacchariden oder synthetischen Polymeren. Bevorzugt wird der Gerüstbildner aus der Gruppe der Polysaccharide ausgewählt. Polysaccharide schließen beispielsweise Homoglykane oder Heteroglykane ein, wie zum Beispiel Alginate, besonders Natriumalginat, Carrageen, Pektine, Tragant, Guar-Gummi, Johannisbrotkernmehl, Agar-Agar, Gummi-Arabikum, Xanthan, natürliche und modifizierte Stärken, Dextrane, Dextrin, Maltodextrine, Chitosan, Glucane, wie ß-1,3-Glucan, ß-1,4-Glucan, wie Cellulose, Mucopolysaccharide, wie Hyaluronsäure etc. Synthetische Polymere schließen beispielsweise ein: Celluloseether, Polyvinylalkohol, Polyvinylpyrrolidon, synthetische Cellulosederivate, wie Methylcellulose, Carboxycellulose, Carboxymethylcellulose, Celluloseester, Cellulosesether wie Hydroxypropylcellulose, Polyacrylsäure, Polymethacrylsäure, Poly(methylmethacrylat) (PMMA), Polymethacrylat (PMA), Polyethylenglykole etc, Es können auch Mischungen mehrerer Gerüstbildner verwendet werden. Erfindungsgemäß besonders bevorzugt sind Alginate, insbesondere Ist Natrium-Alginat bevorzugt. Bevorzugt sind niedrig-viskose Gerüstbildner, Insbesondere calciumfreie Natrium-Alginate, (Natrlum-Alginat mit einem Calciumgehalt < 3 Gew.-%, bevorzugter < 2 Gew.-%, noch bevorzugter < 1,5 Gew.-%), d.h. solche Gerüstbildner, die bevorzugt eine Viskosität von weniger als 2000 mPas, noch bevorzugter von weniger als 1000 mPas, am bevorzugtesten weniger als 100 mPas aufweisen (d.h. eine Lösung von 1 g des Gerüstbildners in 99 ml dest. Wasser (1 %Ige Lösung w/w) bei 20°C und einem pH-Wert von 6-8 hat eine Viskosität von weniger als 2000, bzw, 1000, bzw. 100 mPas). Die Verwendung solcher niedrig-viskoser Gerüstblidner, wie Natrium-Alginat Ist zum einen herstellungsbedingt bevorzugt, zum anderen führt die Anwendung solcher niedrig-viskoser Gerüstbildner zu einer leichteren Löslichkeit der Formulierung bzw. bei Zusatz von Wasser zu einer höheren Zerfalls- bzw. Auflösungsgeschwindigkeit und damit zu einer leichteren Verteilbarkeit auf der Haut. Insbesondere die Verwendung von niedrig-viskosen Alginattypen kann zu einer größeren Auflösungsgeschwindlgkeit der erfindungsgemäß verwendeten Formkörper führen.

Die erfindungsgemäß bevorzugt als Gerüstbildner verwendeten Polysaccharide weisen zweckmäßig durchschnittliche Molmassen von zweckmäßig etwa 10³ bis zu etwa 10⁸, bevorzugt etwa 10⁴ bis 10⁷ auf.

Die Gerüstbildner sind hautverträglich und führen bevorzugt bei der Anwendung auf der Haut zur Ausbildung eines Films, der eine schützende Funktion aufweist.

Klarstellend soll noch erwähnt werden, dass die Formulierung "Gerüstbilder, wobei Proteine ausgenommen sind," Im Sinne der Erfindung nicht die Anwesenheit von Wirkstoffen auf Proteinbasis, wie Enzyme, Hormone etc. ausschließt.

Die erfindungsgemäß verwendeten Formkörper enthalten gegebenenfalls einen oder mehrere Wirkstoffe, bevorzugt mindestens einen Wirkstoff. Wirkstoffe schließen Insbesondere kosmetische oder therapeutische bzw, pharmazeutische, für die äußere Anwendung geeignete Wirkstoffe ein. Bevorzugt enthält der erfindungsgemäß verwendete Formkörper mindestens einen kosmetischen und/oder pharmazeutischen Wirkstoff, Dementsprechend handelt es sich bei den erfindungsgemäß verwendeten Formkörper bevorzugt um kosmetische oder therapeutische Mittel. Kosmetische Formkörper bzw, unter Verwendung kosmetischer Wirkstoffe hergestellte Formkörper Im Sinne der Erfindung sind im wesentlichen Mittel im Sinne des Lebensmittel- und Bedarfsgegenständegesetzes (LMBG), d.h., Stoffe oder Zubereitungen aus Stoffen, die dazu bestimmt sind, äußerlich am Menschen zur Reinigung, Pflege, oder zur Beeinflussung des Aussehens oder des Körpergeruchs, oder zur Vermittlung von Geruchseindrücken angewendet zu werden, es sei, denn, dass sie überwiegend dazu bestimmt sind, Krankheiten, Leiden, Körperschäden oder krankhafte Beschwerden zu lindern oder zu beseitigen. In diesem Sinne handelt es sich bei den erfindungsgemäß verwendeten kosmetischen Formkörpern beispielweise um Badepräparate, Hautwasch- und reinigungsmittel, Hautpflegemittel, insbesondere Gesichtshautpflegemittel, Augenkosmetika, Lippenpflegemittel, Nagelpflegemittel, Fußpflegemittel, Haarpflegemittel, Insbesondere Haarwaschmittel, Haarkonditionierungsmittel, Haarweichspüler etc., Lichtschutzmittel, Hautbräunungsmittel, Depigmentlerungsmittel, Deodorants, Antihydrotika, Haarentfernungsmittel, Insektenrepellents etc. oder derartige Mittel In Kombination.

Beispiele kosmetisch gegebenenfalls auch z.B. dermatologischer, therapeutisch wirksamer Verbindungen schließen ein: Antiaknemittel, antimikrobleile Mittel, Antitranspirationsmittel, adstringierende Mittel, desodorierende Mittel, Enthaarungsmittel, Konditionierungsmittel für die Haut, hautglättende Mittel. Mittel zur Steigerung der Hauthydratation wie z. B. Glycerin oder Harnstoff, Sonnenschutzmittel, Keratolytika, Radikalfänger für freie Radikale, Antiseborrhölka, Antischuppenmittel, antlseptische Wirkstoffe, Wirkstoffe zur Behandlung der Anzeichen der Hautalterung und/oder Mittel, die die Differenzierung und/oder Proliferation und/oder Pigmentierung der Haut modulieren, Vitamine wie Vitamin C, Wirkstoffe mit reizender Nebenwirkung, wie alpha-Hydroxysäuren, ß-Hydroxysäuren, alpha-Ketosäuren, ß-Ketosäuren, Retinoide (Retinol, Retinal, Retin-Säure) Anthrallnen (Dioxyanthranol), Anthranoide, Peroxide (insbesondere Benzoylperoxid), Minoxidil, Lithiumsalze, Antimetabollte, Vitamin D und seine Derivate; Katechlne, Flavonolde, Ceramide, Fettsubstanzen, wie Mineralöle, wie Paraffinöle oder Vaselineöle, Slliconöle, Pflanzenöle wie Kokosöl, Süßmandelöl, Aprikosenöl, Maisöl, Jojobaöl, Olivenöl, Avocadoöl, Sesamöl, Palmöl, Eukalyptusöl, Rosmarinöl, Lavendelöl, Klefernöl, Thymianöl, Minzöl, Kardamonöl, Orangenblütenöl, Sojaöl, Kleleöl, Reisöl, Rapsöl und Rizinusöl, Welzenkelmöl und daraus Isoliertes Vitamin E, Nachtkerzenöl, Pflanzenlecithine (z.B. Sojalecithin), aus Pflanzen Isolierte Sphingolipide/Ceramide, tierische Öle oder Fette, wie Talg, Lanolin, Butteröl, Fettsäureester, Ester von Fettalkoholen und Wachse mit einem der Hauttemperatur entsprechenden Schmelzpunkt (tierische Wachse, wie Bienenwachs, Carnaubawachs und Candeilllawachs, mineralische Wachse, wie mikrokristalline Wachse, und synthetische Wachse, wie Polyethylen- oder Slllkonwachse), sowie sämtliche für kosmetische Zwecke geeigneten Öle, wie beispielsweise In der CTFA-Abhandlung, Cosmetlc Ingredlent Hand-book, 1. Aufig., 1988, The Cosmetic, Toiletry and Fragrance Association, Inc., Washington, erwähnt, mehrfach ungesättigte Fettsäuren, essentielle Fettsäuren (z.B. gamma-Linolensäure), Enzyme, Coenzyme, Enzymhemmstoffe, hydratisierende Mittel, hautberuhigende Mittel, Detergentien oder schaumbildende Mittel, und anorganische oder synthetische mattierende Füllstoffe, abraslve Mittel.

Weiterhin können Pflanzenwirkstoffextrakte bzw. daraus gewonnene Auszüge oder Einzelstoffe erwähnt werden. Allgemein wird der Pflanzenwirkstoffextrakt in der Regel ausgewählt aus der Gruppe bestehend aus festen Pflanzenextrakten, flüssigen Pflanzenextrakten, hydrophilen Pflanzenextrakten, lipophilen Pflanzenextrakten, einzelnen Pflanzenlnhaltsstoffen; sowie deren Mischungen, wie Flavonoide und Ihre Aglyka: Rutin, Quercetin, Diosmin, Hyperosid, (Neo)hesperidin, Hesperitin, Ginkgo Biloba (z.B. Ginkoflavongiykoside), Crataegus-Extrakt (z.B. oligomere Procyanidine), Buchweizen (z.B. Rutin), Sophora Japonica (z.B. Rutin), Birkenblätter (z.B. Quercetinglykoside, Hyperosid und Rutin), Holunderblüten (z.B. Rutin), Lindenblüten (z.B. ätherisches Öl mit Quercetln und Farnesol), Johanniskautöl, (z.B. Olivenölauszug), Calendula, Arnika (z.B. ölige Auszüge der Blüten mit ätherischem Öl, polare Auszüge mit Flavonolden), Mellsse (z.B. Flavone, ätherisches Öl); Immunstimulantien: Echinacea purpurea (z.B.alkoholische Auszüge, Frischpflanzensaft, Preßsaft), Eleutherokokkus senticosus; Alkaloide: Rauwolfia (z.B. Prajmalin), Immergrün (z.B.Vincamin); weitere Phytopharmaka; Aloe, Roßkastanie (z.B. Aescin), Knoblauch (z.B. Knoblauchöl), Ananas (z.B. Bromelaine), Ginseng (z.B. Ginsenoside), Marlendistelfrüchte (z.B. auf Silymarin standardisierter Extrakt), Mäusedornwurzel (z.B. Ruscogenin), Baldrian (z.B. Valepotriate, Tct. Valerianae), Kava-Kava (z.B. Kavalactone), Hopfenblüten (z.B. Hopfenbitterstoffe), Extr. Passi-florae, Enzian (z.B. ethanol. Extrakt), anthrachinonhaltige Drogenauszüge, z.B. aloinhaltiger Aloe Vera-Saft, Pollenextrakt, Algenextrakte, Süßholzwurzelextrakte, Palmenextrakt, Galphimia (z.B.Urtinktur), Mistel (z.B. wässrig-ethanol. Auszug), Phrtosterole (z.B. beta-Sitosterin), Wollblumen (z.B. wäßrig-alkohol. Extrakt), Drosera (z.B. Likörwelnextrakt), Sanddornfrüchte (z.B. daraus gewonnener Saft oder Sanddornöl), Elbischwurzei, Primelwurzelextrakt, Frischpflanzenextrakte aus Malve, Beinwell, Efeu, Schachtelhalm, Schafgarbe, Spitzwegerich (z.B. Preßsaft), Brennessel, Schöllkraut, Petersilie; Pflanzenextrakte aus Norolaena lobata, Tagetes lucida, Teeoma siems, Momordlca charantla, und Aloe Vera Extrakte.

Bevorzugte kosmetische Wirkstoffe sind natürliche und synthetische Feuchthaltefaktoren wie z. B. Glycerin, Harnstoff und Ceramide, Hautschutzmittel, Hautaufheller, Vitamine, Antioxidantien, sogenannte Antiagingmittel, antiirritative Mittel, Sonnenschutzmittel, etc.

Im Unterschied zu den vorstehend beschriebenen im wesentlichen in der Kosmetik verwendeten Formkörpern handelt es sich bei den therapeutisch verwendeten Formkörpern (Arzneimittel) um solche, die mindestens einen pharmazeutischen bzw. therapeutischen insbesondere auch dermatologischen Wirkstoff enthalten und die Im Sinne des Arzneimittelgesetzes unter anderem dazu bestimmt sind, Krankheiten, Leiden, Körperschäden oder krankhafte Beschwerden zu hellen, zu lindern oder zu verhüten. Solche Mittel bzw. Wirkstoffe sind für die äußere Anwendung bestimmt, wobei es sich um hautaktive Wirkstoffe aber auch um transdermale Wirkstoffe handelt kann. Sie schließen beispielsweise ein: Mittel zur Behandlung von Hautkrankheiten, äußerlich anwendbare Analgetika, z. B. Dextropropoxyphen, Pentazocin, Pethidin, Buprenorphin: Antirheumatika/Antiphlogistika (NSAR), z. B. Indometacin, Diclofenac, Naproxen, Ketoprofen, Ibuprofen, Flurbiprofen, Salicylsäure und -derivate wie Acetylsalicylsäure, Oxicame; Steroidhormone, z. B. Betamethason, Dexamethason, Methylprednisoion, Ethinylestradiol, Medroergotamin, Dihydroergotoxin; Gichtmittel, z. B. Benzbromaron, Allopurinol; Dermatika Externa, einschließlich antibakterielle Mittel, Antimykotika, antivirale Wirkstoffe, entzündungshemmende Wirkstoffe, juckreizstillenden Wirkstoffe, anästhesierende Wirkstoffe, z. B. Benzocain, Corticoide, Aknemittel, antiparasitäre Wirkstoffe; äußerlich anwendbare Hormone; Venentherapeutika; Immunsuppresiva etc. alle für die äußerliche Anwendung.

Bevorzugte therapeutische Mittel sind Analgetika, z.B. Immunsuppressiva, Hormone, Mittel zur Behandlung von Hautkrankheiten, wie der Neurodermitis, der atopischen Dermatitis etc., und Anti-Herpesmittel.

Auch die Gerüstblider, Insbesondere die Polysaccharide können gewisse therapeutische Wirkungen aufweisen (so wirkt der bevorzugt verwendete Gerüstbildner (Natrium-)Alginat In gewissen Ausmaß antiviral), sie sind Jedoch keine Wirkstoffe Im Sinne der Erfindung.

Die erfindungsgemäß verwendeten Formkörper enthalten weiterhin gegebenenfalls einen oder mehrere Hilfsstoffe, Hilfsstoffe schließen ein: oberflächenaktive Mittel neben den oben erwähnten Waschtensiden, wie Dispergiermittel, Emulgatoren etc., Füllstoffe, pH-Einstellungsmittel, wie Pufferstoffe, Stabilisatoren, Cosolventlen, pharmazeutisch und kosmetisch gebräuchliche oder sonstige Farbstoffe und Pigmente, Konservierungsmittel, Weichmacher, Schmiermittel bzw. Gieltmittel, etc. Ein besonders bevorzugter Hilfsstoff stellt das Squalan dar.

Die erfindungsgemäßen Formkörper dienen der äußeren Anwendung bei Menschen oder Tieren. Die äußere Anwendung erfolgt dabei so, dass der erfindungsgemäße Formkörper mit Wasser bzw. einer wäßrigen Lösung, die einen oder mehrere Wirkstoffe und/oder Hilfsstoffe enthält, angefeuchtet oder in Wasser gelöst wird. Je nach Flüssigkeitsmenge und Löslichkeit des Formkörpermaterials das verwendet werden, kann der Formkörper vollständig unter Bildung einer Lösung gelöst werden oder unter Bildung eines Gels zerfallen. Wird der erfindungsgemäße Formkörper in einer größeren Wassermenge gelöst, handelt es sich in der Regel um eine Badeanwendung und diese Anwendung ist erfindungsgemäß In der äußeren Anwendung enthalten. Bevorzugt erfolgt die Anwendung Jedoch so, dass die Formkörper mit einer kleinen Menge Wasser oder einer Wirk- und/oder Hilfsstofflösung unter Bildung eines Gels direkt auf der Haut oder Im Haar befeuchtet werden und dort verrieben bzw. einmassiert werden.

Die vorliegende Erfindung betrifft auch eine Kombination, enthaltend mindestens einen der erfindungsgemäß verwendeten Formkörper sowie mindestens eine wässrige Lösung, die einen oder mehrere Wirkstoffe und/oder Hilfsstoffe enthält, in einer zusammengehörenden, räumlichen Anordnung (Anwendungspaket, Set, Klt-of-Parts etc.). Bei der Wirkstofflösung kann es sich z.B. um Lösungen von leicht-flüchtigen Wirk- und/oder Hilfsstoffen handeln, die aufgrund des Herstellverfahrens durch die Gefriertrocknung nicht In den Formkörper eingebracht werden sollen bzw. können, wie z.B. gewisse Anteile ätherischer Öle, Parfums etc.

Je nach vorhandener Menge und Art der zusätzlich vorhandenen Wirkstoffe und/oder Hilfsstoffe enthält der erfindungsgemäß verwendete Formkörper bevorzugt mindestens etwa 10 Gel.-% des Gerüstbildners, bezogen auf das Gesamtgewicht des Formkörpers, bevorzugt mindestens etwa 15, bevorzugter 30, noch bevorzugter mindestens etwa 50 Gew.-% bis zu 100 Gew.-% des Gerüstbildners, besonders Polysaccharide, wie Natriumalginat.

Die Formkörper enthalten im allgemeinen auch Reste von Wasser. Je nach Art des Wirkstoffes [hydrophil, hydrophob) kann der Wassergehalt bei bis zu 20 Gew.-% liegen. Der Wassergehalt kann sich nach der Herstellung des Formkörpers durch Gefriertrocknung bei der Lagerung verändern, in der Regel erhöhen. Bevorzugt liegt der Wassergehalt des Formkörpers nach der Herstellung bei etwa 2 bis 15, bevorzugter bei 2 bis 12 Gew.-%.

Die erfindungsgemäß verwendeten Formkörper enthalten von 0 bis zu 85 Gew.-%, bevorzugt von 0,000001 Gew.-% bis zu 50 Gew.-% eines oder mehrerer Wirkstoffe. Die Mengenverhältnisse hängen sehr stark davon ab, ob es sich bei dem Wirkstoff um einen kosmetischen oder therapeutischen Wirkstoff handelt. Insbesondere die therapeutischen Wirkstoffe kommen unter Umständen in sehr kleine Mengen zur Anwendung.

Die Formkörper enthalten von 0 bis 85 Gew.-% eines oder mehrerer Hilfsstoffe. Die Formkörper können 0,1 bis 70 Gew.-% der Hilfsstoffe enthalten, noch bevorzugter 5 bis 60 Gew.-% Hilfsstoffe.

Insbesondere in der Anwendung mit einer zusätzlichen Wirkstofflösung sind auch Formkörper anwendbar, die lediglich aus dem hydrophilen Gerüstbildner und gegebenenfalls zusätzlichen Hilfsstoffen bestehen.

Ein bevorzugter Hilfsstoff Ist Squalan. Dabei handelt es sich um ein kosmetisches Öl. Das Squalan bewirkt überraschend trotz seiner hydrophoben Beschaffenheit eine verbesserte Löslichkelt des Formkörpers, was die äußere Anwendung auf der Haut erleichtert. Squalan besitzt daneben auch hautpflegende Wirkungen, obwohl nicht Wirkstoff im Sinne der Erfindung. In einer bevorzugten Ausführungsform enthält der Formkörper von etwa 10 bis 60 Gew.-% Squalan (bestimmbar durch Extraktion mit Diethylether nach der Methode von Welbull Stoldt in Amtliche Sammlung von Untersuchungsverfahren ASU nach §35 LMBG)

Die Einordnung der vorstehend erwähnten Stoffe In die Kategorie der Hilfsstoffe im Rahmen der vorliegenden Erfindung schließt nicht aus, dass diese Hilfsstoffe auch gewisse kosmetische und/oder therapeutische Wirkungen entfalten können.

Ein besonders bevorzugter Formkörper enthält:
- mindestens 10 Gew.-% eines oder mehrerer Gerüstblidner, insbesondere Polysaccharide, wie Natriumalginat, insbesondere calciumfreies Natriumalginat, dessen 1-gewichtsprozentige Lösung oder Suspension In Wasser (1 g in 99 ml Wasser bei 20°C, pH 6-8) bevorzugt eine Viskosität von weniger als 2000, bevorzugt weniger als 1000, besonders bevorzugt weniger als 100 mPas aufweist,
- 0,000001 Gew.-% bis zu 50 Gew.-% eines oder mehrerer Wirkstoffe,
- 0,1 bis 70 Gew.-% Gew.-% eines oder mehrerer Hilfsstoffe, wie insbesondere Squalan, sowie
- bis zu 20 Gew.-%, bevorzugt bis zu 15 Gew.-% Wasser,
mit der Maßgabe, dass der Formkörper. keine Proteine als Gerüstbildner aufweist.

Bevorzugt weist der erfindungsgemäß verwendete Formkörper, wie z.B. der der vorstehend erwähnten Zusammensetzung, enthaltend mindestens einen Gerüstbildner, wobei Proteine ausgenommen sind, gegebenenfalls einen oder mehrere Wirkstoffe sowie gegebenenfalls einen oder mehrere Hilfsstoffe, zur äußeren Anwendung,
- eine Dichte von 0,005 g/cm³ bis zu 0,8 g/cm³ bevorzugt 0,01 g/cm³ bis zu 0,8 g/cm³,
- ein Volumen von 0,1 cm³ bis 6 cm³, bevorzugt 0,8 cm³ bis 6 cm³, und
- einen Durchmesser (maximaler Abstand zwischen zwei Punkten des Formkörpers) von mindestens 6 mm auf.

Die erfindungsgemäßen verwendeten Formkörper stellen poröse Formkörper mit homogener Verteilung der Inhaltsstoffe dar (abgesehen von gegebenenfalls vorhandenen Beschichtungen).

Die Auflösungsgeschwindigkeit der erfindungsgemäß verwendeten Formkörper, gemessen entsprechend einer Methode zur Messung der "Zerfallszeit von Tabletten und Kapsein" mit einer Testapparatur nach PharmEU beträgt bevorzugt weniger als 1 Minute (bei Formkörpern mit 9 mm Durchmesser liegt nach < 20 Sekunden komplette Hydratation ohne erkennbaren Kern vor).

Die erfindungsgemäß verwendeten Formkörper sind erhältlich durch ein Verfahren, das die folgenden Schritte umfasst:
(a) Herstellen einer Lösung oder Suspension, die mindestens ein Blopolymeres, ggf. einen oder mehrere physiologisch aktive Wirkstoffe sowie ggf. einen oder mehrere Hilfsstoffe enthält,
(b) Giessen der Lösung in eine Form, die einer geometrischen Kugelform mit einem Durchmesser von mindestens 5 mm entsprechende Hohlräume aufweist,
(c) Gefrieren der Lösung In der Form und
(d) Gefriertrocknung der gefrorenen Lösung unter Bildung des Formkörpers der die geometrische Form einer Kugel und einen Durchmesser von mindestens 5 mm aufweist

Gegebenenfalls können zwischen diesen Schritte weitere Schritte durchgeführt werden, insbesondere Ist es möglich, nach dem Schritt (c) eine Bearbeitung der Oberfläche der Formkörper durch mechanische Bearbeitung oder durch Besprühen mit z.B. Wirkstofflösungen, Farbstofflösungen und/oder die Auflösegeschwindigkeit modifizierende Mitteln durchzuführen. Bevorzugt weist der Formkörper jedoch keine Oberflächenbeschichtung auf und ist homogen, im Sinne einer gleichen Verteilung der Bestandteile über den gesamten Formkörper aufgebaut.

Zweckmäßig geht man bei der Herstellung so vor, dass man zunächst eine wässrige Lösung des Gerüstbildners herstellt und anschließend gegebenenfalls einen oder mehrere Wirkstoffe bzw. einen oder mehrere Hilfsstoffe hinzugibt und vermischt.

Damit dem Formkörper eine ausreichende mechanische Stabilität verliehen werden kann, Ist es erforderlich, dass die Lösung bzw. Suspension eine gewissen Konzentration des Gerüstbildners aufweist. Diese Konzentration hängt natürlich ab von der Art des verwendeten Gerüstbildners. Sie beträgt zweckmäßig etwa mindestens 0,1 Gew.-% bezogen auf die Gesamtmenge der Lösung, bevorzugt mindestens etwa 0,25 Gew.-% bis zu etwa 20 Gew.-%, bevorzugt weniger als 15 Gew.-%, noch bevorzugter weniger als 10 Gew.-% (Gewicht des Gerüstbildners bezogen auf das Gesamtgewicht der Lösung). Höhere Konzentrationen sind nicht bevorzugt, weil dann die Viskosität der Lösung zu hoch wird, und dadurch die Verarbeitbarkeit der Lösung erschwert wird. Die Menge des In der Lösung bzw. Suspension enthaltenden Gerüstbildners beeinflusst maßgeblich die Dichte (Gewicht des Formkörpers bezogen auf das Volumen der geometrischen Form des Formkörpers) des erhaltenen Formkörpers. Die Dichte Ist wiederum eine wichtige Größe für die Auflösungsgeschwindigkeit des Formkörpers beim Befeuchten mit Wasser oder einer Wirk- und/oder Hlifsstofflösung. Je höher die Konzentration des Gerüstbildners In der Lösung ist, umso höher wird die Dichte (umso geringer wird der Porositätsgrad) des Formkörpers und umgekehrt. Unter dem Gesichtspunkt Dichte/Porositätsgrad bzw. der Auflösungsgeschwindigkeit wird die Konzentration des Gerüstbildners In der im Schritt (a) hergestellten Lösung bzw. Suspension bevorzugt aus einem Bereich ausgewählt von etwa 0,25 Gew.-% bis etwa 15 Gew.-%. Die Konzentration des bevorzugt verwendeten Gerüstbildners Natriumalginat beträgt bevorzugt von 0,5 bis 5 Gew.-%, bevorzugt 1 bis 4 Gew.-%.

Die Dichten der erfindungsgemäß verwendeten Formkörper liegen zweckmäßig bei etwa 0,01 g/cm³ bis zu 0,8 g/cm³, bevorzugt etwa 0,015 g/cm³ bis zu 0,5 g/cm³. bevorzugt etwa 0,02 g/cm³ bis zu 0,1 g/cm³. Der Begriff der Dichte, wie er vorliegend verwendet wird, bezeichnet das Gewicht des Formkörpers bezogen auf das Volumen der äußeren geometrischen Form des Formkörpers.

Das Gewicht der einzelnen Formkörper hängt natürlich von seiner Größe ab. Im allgemeinen liegt das Gewicht der einzelnen Formkörper bei etwa 10 bis 200 mg, bevorzugt 20 bis 100 mg. Beispielsweise weisen Kugeln von 12 mm Durchmesser ein Gewicht Im Bereich von bevorzugt 20 bis 80 mg, bevorzugter 30 bis 60 mg auf. Für Kugeln anderer Durchmesser berechnen sich entsprechende Vorzugsbereiche.

Die Herstellung der Lösung, die der Gefriertrocknung unterworfen wird, erfolgt bevorzugt so, dass zunächst eine Lösung des Gerüstbildners hergestellt wird und anschließend in die Lösung des Gerüstblidners die gegebenenfalls vorhandenen Wirkstoffe bzw. Hilfsstoffe eingearbeitet werden. Werden öllösliche Wirkstoffe verwendet, werden diese bevorzugt In gegebenenfalls ais Hilfsstoffe verwendeten Öle (Insbesondere Squalan) gelöst und anschließend der Lösung des Gerüstbildners zugesetzt. Diese Herstellweise besitzt den Vorteil, dass sich stabile Lösungen bzw. Suspensionen bilden. Es werden keine Emulgatoren benötigt, und es findet während der Verarbeitung keine Phasentrennung der Lösung oder Suspension bei Verwendung öllöslicher bzw. öliger Hilfs- bzw. Wirkstoffe statt.

Die so hergestellte Lösung wird dann In eine Form gegossen, die die den Formkörpern entsprechende Hohlräume der gewünschten geometrischen Formen aufweisen. Die Form besteht bevorzugt aus Kautschuk, Silikon-Kautschuk, vulkanisiertem Kautschuk (Gummi) etc. Bevorzugt sind Gummiformen. Die Formmateriallen können gegebenenfalls beschichtet sein. Die Hohlräume der Formkörper, In die die Lösung hineingegossen wird, weisen Im allgemeinen die Form des gewünschten Formkörpers auf. D.h., dass das Volumen des Hohlraums Im wesentlichen dem Volumen der später erhaltenen Formkörper entspricht.

Da das Volumen der in die Hohlräume eingefüllten Lösungen bzw. Suspensionen beim Gefrieren zunimmt (Dichteunterschled zwischen Wasser und Eis), werden die Hohlräume In der Regel nicht vollständig gefüllt. Es werden auf diese Welse vollständig symmetrische Formkörper erhalten. Dies ist beispielsweise nach dem Verfahren durch Eintropfen in tiefkalte Lösungen (wie in flüssigen Stickstoff) nicht möglich, da es dort zu unsymmetrischen Temperaturverteilungen kommt, so dass sich stets mehr oder weniger starke Abweichungen von einer regelmäßigen Form ergeben. Solche unregelmäßig geformten Formkörper sind jedoch gerade im Bereich der kosmetischen Endprodukte nicht erwünscht. Das bedeutet in der Regel, dass diese nach dem Eintropfverfahren hergestellten Formkörper einer mechanischen Nachbearbeitung bedürfen, was nach dem Verfahren, wie es erfindungsgemäß verwendet wird, nicht erforderlich ist. Bei nach dem Eintropfverfahren hergestellten Formkörpern wird eine solche Nachbearbeitung mit zunehmendem Volumen des Formkörpers immer erforderlicher, da bei diesem Verfahren deutliche äußerliche Unregelmäßigkeiten auftreten, die insbesondere bei größeren Formkörpern stärker in Erscheinung treten.

Nach dem Einfüllen der Lösung in die Hohlräume der Form wird die Lösung bzw. Suspension gefroren. Das Abkühlen bzw. Gefrieren der Lösung kann an sich in beliebiger Weise erfolgen. Bevorzugt erfolgt das Abkühlen bei dem erfindungsgemäß verwendeten Verfahren durch Anblasen mit kalter Luft. Weitere Verfahren schließen z.B. das Eintauchen der Formen in flüssige Gase ein, wie z.B. das Eintauchen in flüssigen Stickstoff. Die Abkühlgeschwindigkeit beeinflusst dabei die Größe der gebildeten Eiskristalle. Diese beeinflussen wiederum die Porengrößenvertellung des gebildeten Formkörpers. Werden wenige große Kristalle gebildet, so weist der Formkörper wenige große Poren auf. Werden viele kleine Kristalle gebildet, weist der Formkörper viele kleine Poren auf. Die Kristalle werden um so kleiner, je höher die Abkühlgeschwindigkeit der Lösung bzw. Suspension ist.

Die Gefriertemperatur, die erforderlich ist, hängt unter anderem davon ab, wie stark die Gefrierpunktserniedrigung durch die In der Lösung enthaltenden Wirkstoffe bzw. Hilfsstoffe ist. Zweckmäßig liegt die Temperatur unterhalb des Gefrierpunktes von Wasser bis zur Temperatur von flüssigem Stickstoff (- 196°C). Bevorzugt ist die Gefriertemperatur etwa -20 bis -80°C. Nach dem Gefrieren der Lösung bzw. Suspension werden die Formkörper aus der Form genommen und gegebenenfalls einer Nachbearbeitung unterzogen. Die Nachbearbeitung kann mechanisch erfolgen, z.B. durch eine Oberflächenbehandlung (Schleifen, Aufrauhen etc.). Dasweiteren Ist eine Beschichtungsbehandlung möglich wie z.B. das Besprühen mit einer Salzlösung, z.B. zur Bildung weniger löslicher Formen der Gerüstbildner, insbesondere bei der Verwendung von Natriumalginat und Salzlösungen mehrwertiger Metalllonen. Auch kann eine Farblösung oberflächlich auf die gefrorenen Formkörper aufgebracht werden, die zu gefärbten Formkörpern führt.

Anschließend werden die Formkörper der Gefriertrocknung unterworfen. Die Gefriertrocknung kann in an sich bekannter Welse erfolgen, wie z.B. beschrieben In DE 4328329 C2 oder DE 4028622 C2.

Die Erfindung wird durch die folgenden Beispiele näher veranschaulicht.

### BEISPIELE

### Beispiel 1 (Natriumalginatkugel, Durchmesser 12 mm)

### Basisversion nur Alginat

| | |
|---|---|
| 2 g | Protanal LF 10/60 (Na-Alginat) |
| 98 g | Wasser |

2 g Na-Alginat (Protanal LF 10 / 60) werden unter Rühren In 98 g Wasser gegeben (2 %-Lösung w/w). Die homogene (entgaste) Mischung wird in Formen gegossen, unter Anblasen von Luft ausgefroren, aus der Form genommen und anschließend In an sich bekannter Weise gefriergetrocknet. Es werden ca. 110 Kugeln erhalten.

### Beispiel 2 (Kugel, Durchmesser 12 mm)

### Alginat mit Squalan

| | |
|---|---|
| 2 g | Protanal LF 10 / 60 (Na-Alginat) |
| 1 g | Squalane |
| 97 g | Wasser |

2 g Na-Alginat werden unter Rühren In 97 g Wasser gegeben und homogen gemischt. Anschließend wird unter Rühren 1 g Squalan hinzugegeben. Die homogene (entgaste) Mischung wird In Formen gegossen, unter Anblasen von Luft ausgefroren, aus der Form genommen und anschließend In an sich bekannter Weise gefriergetrocknet.

### Beispiel 3 (Kugel, Durchmesser 9 mm)

### Alginat mit Squalan und Glycerin

| | |
|---|---|
| 2,0 g | Propanal LF 10 / 60 (Na-Alginat) |
| 0,9 g | Squalan |
| 0,6 g | Glycerin |
| 96,5 g | Wasser |

2 g Na-Alginat werden unter Rühren In 96,5 g Wasser aufgelöst. Anschließend wird unter Rühren eine Mischung aus 0,9 g Squalan und 0,6 g Glycerin hinzugegeben. Die homogene (entgaste) Mischung wird In Formen gegossen, unter Anblasen von Luft ausgefroren, aus der Form genommen und anschließend In an sich bekannter Weise gefriergetrocknet.

### Beispiel 4: (Kugel, Durchmesser 12 mm)

### Alginat mit Squalan und Harnstoff

| | |
|---|---|
| 2,0 g | Protanal LF 10 / 60 (Na-Alginat) |
| 1,0 g | Squalan |
| 1,0 g | Harnstoff |
| 96,0 g | Wasser |

2 g Na-Alginat und 1,0 g Harnstoff werden unter Rühren In 96,0 g Wasser aufgelöst. Anschließend wird unter Rühren 1,0 g Squalan hinzugegeben. Die homogene (entgaste) Mischung wird In Formen gegossen, unter Anblasen von Luft ausgefroren, aus der Form genommen und anschließend in an sich bekannter Weise gefriergetrocknet.

### Beispiel 5: (Kugel, Durchmesser 12 mm)

### Alginat mit Squalane und Ceramiden

| | |
|---|---|
| 2,0 g | Protanal LF 10 / 60 (Na-Alginat) |
| 1,5 g | Squalan |
| 0,01 g | Ceramid |
| 96,4 g | Wasser |

2 g Na-Alginat werden unter Rühren In 96,4 g Wasser aufgelöst. Anschließend wird unter Rühren eine Mischung aus 1,5 g Squalan und 0,01 g Ceramide hinzugegeben. Die homogene (entgaste) Mischung wird In Formen gegossen, unter Anblasen von Luft ausgefroren, aus der Form genommen und anschließend In an sich bekannter Weise gefriergetrocknet.

## Patentansprüche

1. Gefriergetrockneter Formkörper zur äußeren Anwendung, enthaltend mindestens einen Gerüstbildner, wobei Proteine ausgenommen sind, gegebenenfalls einen oder mehrere Wirkstoffe sowie gegebenenfalls einen oder mehrere Hilfsstoffe, der **dadurch gekennzeichnet ist, dass** er die geometrische Form einer Kugel mit einem Durchmesser von mindestens 5 mm und eine Auflösungsgeschwindigkeit von weniger als 1 Minute, entsprechend der Methode zur Messung der "Zerfallszeit von Tabletten und Kapseln" mittels PharmEU Testapparatur, aufweist.

2. Gefriergetrockneter Formkörper nach Anspruch 1, der eine Dichte von 0,01 g/cm³ bis 0,8 g/cm³ aufweist.

3. Gefriergetrockneter Formkörper nach Anspruch 1 oder 2, worin der Gerüstbildner ein niedrig-viskoser Gerüstbildner ist, dessen 1 gewichtsprozentige Lösung oder Suspension In Wasser bei 20°C und pH 6-8 eine Viskosität von weniger als 2000 mPas aufweist.

4. Gefriergetrockneter Formkörper nach einem der Ansprüche 1 bis 3, worin der Gerüstbildner ein Polysaccharid oder ein Derivat davon, ein synthetisches Polymer oder eine Mischung davon ist.

5. Gefriergetrockneter Formkörper nach einem der Ansprüche 1 bis 4, worin der Gerüstbildner Natrium-Alginat mit einem Calciumgehalt < 3 Gew.-%, eine natürliche oder modifizierte Stärke oder ein synthetisches Polymer aus der Gruppe umfassend Polyacrylsäure, Polymethacrylsäure, Polymethacrylat PMA, synthetische Cellulosederivate und Celluloseether ist.

6. Gefriergetrockneter Formkörper nach einem der Ansprüche 1 bis 5, worin der Formkörper ein Volumen von 0,1 cm³ bis 6 cm³ aufweist.

7. Gefriergetrockneter Formkörper nach einem der Ansprüche 1 bis 6, der erhältlich Ist durch Gefriertrocknung einer in Form einer Kugel mit einem Durchmesser von mindestens 5 mm gefrorenen Lösung oder Dispersion, enthaltend mindestens einen Gerüstbildner, wobei Proteine ausgenommen sind, gegebenenfalls einen oder mehrere Wirkstoffe sowie gegebenenfalls einen oder mehrere Hilfsstoffe.

8. Gefriergetrockneter Formkörper nach einem der Ansprüche 1 bis 6, der erhältlich ist durch ein Verfahren, das die Schritte umfasst:
a. Herstellen einer Lösung oder Suspension, die mindestens einen Gerüstbildner, wobei Proteine ausgenommen sind" gegebenenfalls einen oder mehrere physiologisch aktive Wirkstoffe sowie gegebenenfalls einen oder mehrere Hilfsstoffe enthält,
b. Giessen der Lösung in eine Form, die einer geometrischen Kugelform mit einem Durchmesser von mindestens 5 mm entsprechende Hohlräume aufweist,
c. Gefrieren der Lösung in der Form und
d. Gefriertrocknung der gefrorenen Lösung unter Bildung des Formkörpers der die geometrische Form einer Kugel und einen Durchmesser von mindestens 5 mm aufweist.

9. Gefriergetrockneter Formkörper der erhältlich ist durch ein Verfahren gemäß Anspruch 8, worin die Konzentration des Gerüstbildners In Schritt a.) mindestens 0,1 Gew.-% bis zu 20 Gew.-%, jeweils bezogen auf das Gesamtgewicht der Lösung, beträgt.

10. Gefriergetrockneter Formkörper nach einem der Ansprüche 1 bis 9, enthaltend:
- mindestens 10 Gew.-% eines oder mehrerer Gerüstbildner, dessen 1 gewichtsprozentige Lösung oder Suspension in Wasser bei 20°C und pH 6-8, eine Viskosität von weniger als 2000 mPas aufweist,
- 0,000001 Gew.-% bis zu 50 Gew.-% eines oder mehrerer Wirkstoffe,
- 0,1 Gew.-% bis 70 Gew.-% eines oder mehrerer Hilfsstoffe, sowie
- bis zu 20 Gew.-% Wasser,
mit der Maßgabe, dass der Formkörper keine Proteine als Gerüstblidner enthält und die geometrische Form einer Kugel mit einem Durchmesser von mindestens 5 mm aufweist.

11. Gefriergetrockneter Formkörper nach einem oder mehreren der Ansprüche 1 bis 10, der einen oder mehrere pharmazeutische oder kosmetische Wirkstoffe oder Hilfsstoffe enthält, die ausgewählt sind aus der Gruppe umfassend Mittel zur Behandlung von Hautkrankheiten wie Dermatika Externa, einschließlich antibakterielle Mittel, entzündungshemmende, juckreizstillende und anästhesierende Wirkstoffe sowie Mittel zur Behandlung von Neurodermitis oder atopischer Dermatitis, Hautaufheller, Antioxidantien, antiirritative Mittel, Vitamine, kosmetische Öle, Glycerin, Harnstoff und Squalan.

12. Gefriergetrockneter Formkörper nach einem der Ansprüche 1 bis 11 zur äußeren therapeutischen Anwendung.

13. Kombination, enthaltend mindestens einen Formkörper wie in einem der Ansprüche 1 bis 12 definiert sowie mindestens eine wässrige Lösung, die einen oder mehrere Wirkstoffe und/oder Hilfsstoffe enthält, in zusammengehöriger, räumlicher Anordnung.

14. Verwendung eines gefriergetrockneten Formkörpers nach einem der Ansprüche 1 bis 11 zur äußeren kosmetischen Anwendung.

## Claims

1. Freeze-dried shaped article for external use, containing at least one skeleton-forming agent, proteins being excepted, optionally one or more active substances and also optionally one or more auxiliary substances, which is **characterized by** having the geometrical shape of a sphere with a diameter of at least 5 mm and by exhibiting a dissolution rate of less than 1 minute, according to the method for measuring the *"Disintegration-time of tablets and capsules"* with a test apparatus according to *PharmEU.*

2. Freeze-dried shaped article according to claim 1 having a density from 0.01 g/cm³ up to 0.8 g/cm³.

3. Freeze-dried shaped article according to claim the 1 or 2, wherein the skeleton-forming agent is a low-viscosity skeleton-forming agent, the 1 per-cent-by-weight solution or suspension of which in water at 20°C and at pH 6-8 has a viscosity of less than 2000 mPas.

4. Freeze-dried shaped article according to one of claims 1 to 3, wherein the skeleton-forming agent is a polysaccharide or a derivative thereof, synthetic polymer or mixtures thereof.

5. Freeze-dried shaped article according to one of claims 1 to 4, wherein the skeleton-forming agent is sodium alginate with a calcium content < 3 wt.%, a natural or modified starch or a synthetic polymer from the group comprising polyacrylic acid, polymethacrylic acid, polymethacrylate PMA, synthetic cellulose derivatives and cellulose ethers.

6. Freeze-dried shaped article according to one of claims 1 to 5, wherein the shaped article has a volume of 0.1 cm³ to 6 cm³.

7. Freeze-dried shaped article according to one of claims 1 to 6, which is obtainable by freeze drying of a frozen solution or dispersion in the shape of a sphere with a diameter of at least 5 mm, containing at least one skeleton-forming agent, proteins being excepted, optionally one or more active substances and also optionally one or more auxiliary substances.

8. Freeze-dried shaped article according to one of claims 1 to 6, which is obtainable by means of a process that comprises the following steps:
(a) producing a solution or suspension that contains at least one scaffold-forming agent, proteins being excepted, optionally one or more physiologically effective active substances and also optionally one or more auxiliary substances,
(b) pouring the solution into a mold that exhibits cavities having the geometrical shape of a sphere with a diameter of at least 5 mm,
(c) freezing the solution in the mold and
(d) freeze drying the frozen solution, thereby forming the shaped article having the geometrical shape of a sphere with a diameter of at least 5 mm.

9. Freeze-dried shaped article which is obtainable by the process according to claim 8,
wherein the concentration of the skeleton-forming agent in step a) amounts to at least 0.1 wt.% up to 20 wt.%, relative to the total weight quantity of the solution.

10. Freeze-dried shaped article according to one of claims 1 to 9, containing:
- at least 10 wt.% of one or more skeleton-forming agents, the 1 per-cent-by-weight solution or suspension of which in water at 20°C and at pH 6-8 has a viscosity of less than 2000 mPas,
- 0.000001 wt.% up to 50 wt.% of one or more active substances,
- 0.1 wt.% to 70 wt.% of one or more auxiliary substances,
- and also up to 20 wt.% water,
with the proviso that the shaped article does not comprise any proteins by way of skeleton-forming agents and exhibit the geometrical shape of a sphere with a diameter of at least 5 mm.

11. Freeze-dried shaped article according to one or more of claims 1 to 10, containing one or more pharmaceutically or cosmetically acceptable active substances or auxiliary substances, which are selected from the group comprising agents for the treatment of skin diseases such as external dermatological agents, including antibacterial agents, anti-inflammatory active substances, antipruritic and anaesthetizing active substances, as well as agents for the treatment of neurodermatitis or atopic dermatitis, skin-lighteners, antioxidants, anti-irritative agents, vitamins, cosmetic oils, glycerin, urea and squalane.

12. Freeze-dried shaped article according to one of claims 1 to 11 for external therapeutic application.

13. A combination containing at least one shaped article as defined in one of claims 1 to 12 and also at least one aqueous solution that contains one or more active substances and/or auxiliary substances, in matching spatial arrangement.

14. Use of a freeze-dried shaped article according to one of claims 1 to 11 for external cosmetic application.

## Revendications

1. Corps moulé lyophilisé pour une application externe contentant au moins un agent de structuration, des protéines étant exclues, optionnellement au moins un ou plusieurs agents actifs et optionnellement au moins un ou plusieurs adjuvants, qui est **caractérisé en ce qu'**il a une forme géométrique d'une sphère ayant un diamètre d'au moins 5 mm et une vélocité de dissolution inférieure à 1 minute selon la méthode de mesure du « temps de désintégration des comprimés et des capsules » par un appareil de test selon PharmEU.

2. Corps moulé lyophilisé selon la revendication 1, qui présente une densité de 0,01 g/cm³ à 0,8 g/cm³.

3. Corps moulé lyophilisé selon l'une des revendications 1 ou 2, dans lequel l'agent de structuration est un agent de structuration de basse viscosité, sa solution ou suspension de 1 pourcent en poids dans de l'eau à 20 °C et un pH de 6 à 8 présentant une viscosité inférieure à 2000 mPas.

4. Corps moulé lyophilisé selon l'une des revendications 1 à 3, dans lequel l'agent de structuration est un polysaccaride ou son dérivé, un polymère synthétique ou un mélange de ceux-ci.

5. Corps moulé lyophilisé selon l'une des revendications 1 à 4, dans lequel l'agent de structuration est un alginate de sodium ayant une teneur en calcium < 3 % en poids, un amidon natif ou modifié ou un polymère synthétique du groupe comprenant de l'acide polyacrylique, de l'acide polyméthacrylique, du polyméthacrylate (PMA), des dérivés de cellulose synthétiques et des éthers de cellulose.

6. Corps moulé lyophilisé selon l'une des revendications 1 à 5, dans lequel le corps moulé présent un volume de 0,1 cm³ à 6 cm³.

7. Corps moulé lyophilisé selon l'une des revendications 1 à 6, qui est susceptible d'être obtenu par une lyophilisation d'une solution ou dispersion gelé comprenant au moins un agent de structuration, des protéines étant exclues, optionnellement au moins un ou plusieurs agents actifs et optionnellement au moins un ou plusieurs adjuvants sous une forme d'une sphère ayant un diamètre d'au moins 5 mm.

8. Corps moulé lyophilisé selon l'une des revendications 1 à 6, qui est susceptible d'être obtenu par un procédé comprenant les étapes :
a. la préparation d'une solution ou suspension qui comprend au moins un agent de structuration, des protéines étant exclues, optionnellement au moins un ou plusieurs agents actifs physiologiquement efficaces et optionnellement au moins un ou plusieurs adjuvants,
b. le coulage de la solution dans un moule, qui présente des cavités correspondant à une forme géométrique d'une sphère ayant un diamètre d'au moins 5 mm,
c. la congélation de la solution dans le moule et
d. la lyophilisation de la solution gelée ainsi former le corps moulé qui présente la forme géométrique d'une sphère et un diamètre d'au moins 5 mm.

9. Corps moulé lyophilisé qui est susceptible d'être obtenu par un procédé selon la revendication 8, dans lequel la concentration de l'agent de structuration dans l'étape a.) est au moins 0,1 % en poids à 20 % en poids relatif au poids total de la solution.

10. Corps moulé lyophilisé selon l'une des revendications 1 à 9, contenant :
- au moins 10 % en poids d'un ou plusieurs agents de structuration, sa solution ou suspension de 1 pourcent en poids dans de l'eau à 20 °C et un pH de 6 à 8 présentant une viscosité inférieure à 2000 mPas,
- 0,000001 % en poids à 50 % en poids d'un ou plusieurs agents actifs,
- 0,1 % en poids à 70 % en poids d'un ou plusieurs adjuvants et
- jusqu'à 20 % en poids de l'eau,
à la condition que le corps moulé ne contient pas des protéines comme agent de structuration et présente la forme géométrique d'une sphère ayant un diamètre d'au moins 5 mm.

11. Corps moulé lyophilisé selon l'une des revendications 1 à 10 qui contient un ou plusieurs agents actifs pharmaceutiques ou cosmétiques ou adjuvants qui sont sélectionnés parmi le groupe comprenant des agents pour le traitement des maladies de la peau comme des agents dermatologiques externes incluant des agents antibactériens, des agents anti-inflammatoires, anti-prurigineux et anesthésiants et des agents pour le traitement de la névrodermite ou de la dermatite atopique, des agents de décoloration de la peau, des antioxydants, des agents anti-irritatifs, des vitamines, des huiles cosmétiques, de la glycérine, de l'urée et du squalane.

12. Corps moulé lyophilisé selon l'une des revendications 1 à 11 pour une application externe thérapeutique.

13. Combinaison contentant au moins un corps moulé selon l'une des revendications 1 à 12 et au moins une solution aqueuse qui contient un ou plusieurs agents actifs et /ou adjuvants dans un arrangement assorti spatial.

14. Utilisation d'un corps moulé lyophilisé selon l'une des revendications 1 à 11 pour une application externe cosmétique.
